# EUROPÄISCHE PATENTSCHRIFT

(11) **EP 2 887 860 B1**
(45) Veröffentlichungstag und Bekanntmachung des Hinweises auf die Patenterteilung: **29.07.2020**
(21) Anmeldenummer: 13774080.9
(22) Anmeldetag: 23.08.2013
(51) Int. Cl.: A61B 5/00

(54) **SENSOREINRICHTUNG ZUR DETEKTION BIOELEKTRISCHER SIGNALE**
SENSOR DEVICE FOR DETECTION OF BIOELECTRICAL SIGNALS
DISPOSITIF CAPTEUR POUR LA DÉTECTION DE SIGNAUX BIOÉLECTRIQUES

(30) Priorität: 24.08.2012 DE 102012107838
(43) Veröffentlichungstag der Anmeldung: 01.07.2015
(73) Patentinhaber: CorTec GmbH, 79108 Freiburg (DE)
(72) Erfinder: SCHÜTTLER, Martin, 79312 Emmendingen (DE); RICKERT, Jörn, 79104 Freiburg (DE); HENLE, Christian, 79110 Freiburg (DE); BALL, Tonio, 79117 Freiburg (DE); RAAB, Markus, 79102 Freiburg (DE)
(74) Vertreter: 2s-ip Schramm Schneider Bertagnoll Patent- und Rechtsanwälte Part mbB
(86) Internationale Anmeldenummer: PCT/EP2013/067531
(87) Internationale Veröffentlichungsnummer: WO 2014/029865

(56) Entgegenhaltungen:
- EP-A1- 2 446 921
- DE-U1-202010 015 346
- US-A- 5 178 161
- US-A- 5 715 821
- US-A1- 2012 123 232
- US-B1- 6 597 954

## Beschreibung

### Gebiet der Erfindung

Die Erfindung betrifft eine Sensoreinrichtung zur Detektion bioelektrischer Signale, insbesondere zur Implantation in oder auf ein Gehirn, insbesondere menschliches Gehirn.

### Stand der Technik und Hintergrund der Erfindung

Es ist bekannt, bioelektrische Signale, etwa eines menschlichen Gehirns, mit Hilfe eines medizinischen Implantats zu detektieren bzw. aufzuzeichnen. Zur Detektion bioelektrischer Signale sind mindestens zwei elektrische Kontakte bzw. Elektroden notwendig, die an den biologischen Organismus angeschlossen werden bzw. mit dem biologischen Organismus gekoppelt werden. Mit einem der beiden Kontakte wird das bioelektrische Signal in Form einer elektrischen Potenzialveränderung detektiert. Eine Detektion einer elektrischen Potenzialveränderung wird auch als "Ableiten eines Signals" bezeichnet. Der andere der beiden Kontakte wird mit einem ruhenden, möglichst nicht veränderlichen elektrischen Potenzial im selben Organismus gekoppelt, sodass dieser Kontakt ein sogenanntes Referenzpotenzial bereitstellt. Die Potenzialdifferenz zwischen den beiden elektrischen Kontakten bzw. die Änderung dieser Potenzialdifferenz bildet das zu detektierende bioelektrische Signal. Diese Art der Detektion bzw. Ableitung wird als monopolare Ableitung bezeichnet. Die monopolare Ableitung wird verwendet, wenn das bioelektrische Signal an nur einem einzelnen Kontakt detektiert werden soll.

Als Alternative zur monopolaren Ableitung ist auch die sogenannte bipolare Ableitung bekannt. Bei der bipolaren Ableitung kann die Referenzelektrode, die bei der monopolaren Ableitung ein Referenzpotenzial bereitstellt, auch im elektrisch aktiven Bereich des biologischen Organismus angeordnet werden. Alternativ hierzu ist es bekannt, die Referenzelektrode wie bei der monopolaren Ableitung im elektrisch passiven Bereich des biologischen Organismus anzuordnen und einen dritten elektrischen Kontakt vorzusehen, der im elektrisch aktiven Bereich angeordnet wird. Bei der bipolaren Ableitung mit zwei elektrischen Kontakten und einer Referenzelektrode wird die Potenzialdifferenz zwischen den beiden elektrischen Kontakten bzw. zwischen den beiden elektrischen Kontakten und der Referenzelektrode gemessen.

Die vorliegende Erfindung bezieht sich auf die monopolare Ableitung von bioelektrischen Signalen.

Der elektrische Kontakt, der im elektrisch aktiven Bereich des biologischen Organismus angeordnet ist, wird nachfolgend auch als Ableitkontakt bzw. Ableitelektrode bezeichnet. Der elektrische Kontakt, der ein Referenzsignal bzw. ein Referenzpotenzial bereitstellt, wird nachfolgend als Referenzkontakt bzw. Referenzelektrode bezeichnet.

Die aus dem Stand der Technik bekannte monopolare Ableitung ist jedoch nachteilig, denn die Anordnung der Ableitelektrode und Referenzelektrode relativ zueinander sollte zwei Anforderungen erfüllen, die allerdings weitgehend widersprüchlich sind.

Gemäß einer ersten Anforderung sollen die Ableitelektrode und die Referenzelektrode möglichst dicht beieinander liegen.

Eine möglichst geringe bzw. minimale Distanz zwischen der Ableitelektrode und der Referenzelektrode geht mit einer geringen elektrischen Impedanz zwischen den beiden Elektroden einher, denn geringere Volumina biologischen Gewebes zwischen den beiden Kontakten stellen einen geringeren elektrischen Widerstand dar, als große Volumina. Proportional zum elektrischen Widerstand zwischen den beiden Elektroden entwickelt sich die Amplitude eines thermischen Rauschens, was die Qualität des abzuleitenden bzw. zu detektierenden bioelektrischen Signals negativ beeinflusst bzw. kompromittiert.

Darüber hinaus vermindert ein kleiner Abstand zwischen Ableitelektrode und Referenzelektrode die Fläche einer virtuellen Leiterschleife, die von den Elektrodenzuleitungen und dem Strompfad durch das biologische Gewebe (zwischen beiden Elektrodenkontakten) gebildet wird. Je größer die Fläche einer solchen Leiterschleife ist, desto größer ist die elektrische Spannung zwischen der Ableitelektrode und der Referenzelektrode, die sich durch Induktion bildet, wenn die Leiterschleife von einem magnetischen Wechselfeld durchströmt wird. Diese elektrische Spannung überlagert das zu detektierende bioelektrische Signal und kann es im ungünstigsten Fall vollständig maskieren oder elektronische Verstärker in den Sättigungsbereich bringen, was eine gegebenenfalls notwendige Verstärkung des schwachen bioelektrischen Signals verhindert. Quellen für ein magnetisches Wechselfeld können beispielsweise ein Netzstrom von 50 Hz, eine Diebstahl-Sicherungsanlage in Kaufhäusern, Radiofrequenz-Identifikationssysteme (RFID), oder dergleichen sein.

Gemäß einer zweiten Anforderung sollten die Ableitelektrode und die Referenzelektrode möglichst weit voneinander entfernt angeordnet sein.

Durch einen großen geometrischen Abstand zwischen der Ableitelektrode und der Referenzelektrode kann gewährleistet werden, dass das elektrische Potenzial der Referenzelektrode unabhängig von der Quelle des bioelektrischen Signals in der Nähe der Ableitelektrode ist. Dadurch kann die Potenzialdifferenz zwischen der Ableitelektrode und der Referenzelektrode maximiert werden, sodass das bioelektrische Signal besser detektiert und ausgewertet werden kann und leichter von Störeinflüssen unterschieden werden kann.

Bei einer Referenzelektrode, die nahe an der Ableitelektrode angeordnet ist, entspricht die Charakteristik der Signalableitung einer bipolaren Ableitung, sodass dieser Ansatz zu einer Vermischung der bioelektrischen Signale, die von den beiden Kontakten abgeleitet werden, führt und im ungünstigsten Fall eine Detektion eines bioelektrischen Ereignisses verhindern. Denn wenn beide Kontakte (Referenzelektrode und Ableitelektrode) zeitgleich ein identisches bzw. nahezu identisches Signal detektieren ist die zu verstärkende elektrische Potenzialdifferenz Null bzw. nahezu Null.

Im Stand der Technik wird daher die Referenzelektrode in einiger Entfernung zur Ableitelektrode angeordnet, um eine gute Signalqualität und eine gute örtliche Selektivität zu erreichen, was jedoch voraussetzt, dass das Umfeld sehr störungsarm ist, was in den meisten Fällen nur unter Laborbedingungen möglich ist.

US 2012/0123232 betrifft ein Verfahren und eine Vorrichtung zum Bestimmen von Herzschlagänderung unter Verwendung der Wavelet-Transformation. Es wird ein Netzwerk zum Anlegen eines Bias an Eingänge eines Signalverstärkers vorgeschlagen.

EP 2 446 921 betrifft eine Auswahlschaltung für eine Elektrodenanordnung mit einer Signalschnittstelle sowie ein Verfahren zum Herstellen einer Elektrodenanordnung.

US 6,597,954 betrifft ein System und ein Verfahren zum Kontrollieren epileptischer Anfälle. Eine implantierbare Vorrichtung umfasst ein Stimulationssubsystem, das mit einer Stimulationselektrode gekoppelt ist, um elektrische Hirnstimulation in Antwort auf eine Ereignis bereitzustellen, das von einem Detektionssubsystem empfangen wird, welches sich an einer anderen Stelle des Hirns angeordnet ist.

DE 20 2010 015 346 betrifft eine Elektrode mit Leiterbahnen in Zickzackform.

US 5,715,821 betrifft ein neuronales Netzwerk mit Elektroden und Referenzsensoren.

US 5,178,161 betrifft eine Mikroelektrode gemäß dem Oberbegriff der unabhängigen Ansprüche.

US2009/0033333 betrifft eine Vorrichtung zum Messen von Parametern eines elektrochemischen Prozesses, bei welcher ein Widerstand zwischen zwei Elektroden vorgesehen ist, über welchen auch eine Messeinheit vorgesehen ist.

### Aufgabe der Erfindung

Aufgabe der vorliegenden Erfindung ist es daher, Lösungen zum Ableiten bzw. zur Detektion bioelektrischer Signale, insbesondere bioelektrischer Signale eines Gehirns, bereitzustellen, welche die aus dem Stand der Technik bekannten Nachteile zumindest teilweise vermeiden und welche eine Detektion von bioelektrischen Signalen unabhängig von der Anordnung der Elektroden relativ zueinander ermöglicht und insbesondere die aus US 5,178,161 bekannte Mikroelektrode verbessert.

### Erfindungsgemäße Lösung

Diese Aufgabe wird erfindungsgemäß durch eine Sensoreinrichtung zur Detektion bioelektrischer Signale, zur Implantation in oder auf ein Gehirn, nach Anspruch 1 gelöst. Vorteilhafte Ausgestaltungen und Weiterbildungen der Erfindung sind in den abhängigen Ansprüchen angegeben.

Bereitgestellt wird eine Sensoreinrichtung zur Detektion bioelektrischer Signale, insbesondere zur Implantation in oder auf ein Gehirn, wobei die Sensoreinrichtung eine erste Anzahl von Elektroden, die mit einem biologischen Organismus, insbesondere Nervenzellen eines Nervensystems koppelbar sind, zum Abgreifen einer ersten Anzahl elektrischer Potenziale an dem biologischen Organismus umfasst. Erfindungsgemäß umfasst die Sensoreinrichtung Mittel zum Bereitstellen eines gemittelten elektrischen Referenzpotenzials. Das gemittelte elektrische Referenzpotenzial wird aus mehreren detektierten elektrischen Potenzialen gebildet, die an dem biologischen Organismus abgegriffen werden.

Damit wird gewährleistet, dass die zu verstärkende elektrische Potenzialdifferenz nicht Null ist und auch bei ungünstigen Umgebungsbedingungen ein bioelektrisches Ereignis zuverlässig detektiert werden kann. Gegenüber der aus dem Stand der Technik bekannten monopolaren Ableitung zeichnet sich die erfindungsgemäße Sensoreinrichtung durch ein vermindertes Rauschen und große Signalamplituden aus, was zu einer verbesserten Signalqualität führt. Ein weiterer Vorteil ist die verminderte Störempfindlichkeit, beispielsweise gegenüber Einkopplungen von elektromagnetischen Interferenzen, was zu einer verbesserten Zuverlässigkeit der erfindungsgemäßen Sensoreinrichtung führt.

Vorteilhaft ist es, dass die Mittel zum Bereitstellen eines gemittelten elektrischen Referenzpotenzials in die Sensoreinrichtung integriert sind und mit der Sensoreinrichtung implantierbar sind.

Dadurch kann die an eine körperexterne Verarbeitungseinheit zu übertragende Informationsmenge erheblich reduziert werden, weil nur mehr ein gemitteltes elektrisches Referenzpotenzial übertragen werden muss und nicht mehrere einzelne Referenzpotenziale, die von der körperexternen Verarbeitungseinheit gemittelt werden müssten.

Die Sensoreinrichtung umfasst erfindungsgemäß eine erste Anzahl von Differenzverstärkern , wobei ein erster Eingang eines jeden Differenzverstärkers jeweils mit einer Elektrode der ersten Anzahl von Elektroden und ein zweiter Eingang mit dem Mittel zum Bereitstellen eines gemittelten elektrischen Referenzpotenzials verbunden sind.

Damit kann in vorteilhafter Weise eine Änderung der Potenzialdifferenz zwischen einer Elektrode und dem gemittelten elektrischen Referenzpotenzial zu einer elektrisch leicht handhabbaren elektrischen Größe verstärkt werden.

Die Referenzelektrode, die aus der zweiten Anzahl von Referenzelektroden gebildet wird, kann so in unmittelbarer Nachbarschaft zu den Ableitelektroden angeordnet werden, sodass die Vorteile zweier möglichst dicht beieinander liegender Elektroden voll ausgenutzt werden können. Durch Bilden der Referenzelektrode aus einer Anzahl von Referenzelektroden wird verhindert, dass die Ableitcharakteristik einer bipolaren Ableitung entspricht. Die zweite Anzahl von Referenzelektroden kann örtlich nahe um eine Ableitelektrode verteilt angeordnet werden.

Jede Referenzelektrode der zweiten Anzahl von Referenzelektroden detektiert ein elektrisches Potenzial in ihrer unmittelbaren Nähe. Durch das galvanische bzw. elektrische Verbinden der einzelnen Referenzelektroden der zweiten Anzahl von Referenzelektroden werden die einzelnen detektierten Potenziale gemittelt. Dadurch wird das Referenzpotenzial unempfindlich gegen lokale bioelektrische Signale, wobei die Referenzelektrode bzw. die zweite Anzahl von Referenzelektroden dennoch in direkter Nachbarschaft zur ersten Anzahl von Elektroden bzw. zu den Ableitelektroden angeordnet werden kann.

Die zu verstärkende Potenzialdifferenz zwischen dem gemittelten elektrischen Referenzpotenzial und einem elektrischen Potenzial an der Ableitelektrode ist damit groß und der Einfluss von Störgrößen, etwa thermisches Rauschen und magnetisch induzierte Spannung kann reduziert bzw. minimiert werden.

Die galvanischen Verbindungen zwischen den Referenzelektroden der zweiten Anzahl von Referenzelektroden kann eine mechanisch dehnbare Verbindung umfassen. Dadurch wird verhindert, dass eine mechanische Dehnung eines Implantats, auf dem die Elektroden und die Leiterbahnen angeordnet sind, zu einem Leiterbahnbruch führen.

Als vorteilhaft hat sich herausgestellt, wenn die galvanischen Verbindungen zwischen den Referenzelektroden mäanderförmige Leiterbahnen umfassen.

In einer vorteilhaften Ausgestaltung der Erfindung sind die Referenzelektroden so relativ zu den Elektroden (Ableitelektroden) angeordnet, dass die Abstände zwischen einer Referenzelektrode und den direkt benachbarten Elektroden im Wesentlichen gleich groß sind.

In einer Ausgestaltung der Erfindung kann die erste Anzahl von Elektroden größer sein als die zweite Anzahl von Referenzelektroden.

In einer zweiten Alternative der Erfindung umfassen die Mittel zum Bereitstellen eines gemittelten elektrischen Referenzpotenzials eine erste Anzahl von ersten elektrischen Widerständen, wobei jede Elektrode mit jeweils einen der ersten elektrischen Widerstände verbunden ist und die ersten elektrischen Widerstände ihrerseits in einem Knotenpunkt miteinander verbunden sind, der das gemittelte elektrische Referenzpotenzial bereitstellt.

Das elektrische Referenzpotenzial wird durch die an den Ableitelektroden detektierten elektrischen Potenziale gebildet, sodass keine Referenzelektroden zum Bereitstellen eines Referenzpotenzials vorgesehen werden müssen. Damit können sämtliche Elektroden zum Ableiten von bioelektrischen Signalen verwendet werden.

Der Knotenpunkt kann über einen zweiten elektrischen Widerstand mit dem Massepotenzial der Versorgungsspannung der Differenzverstärker verbunden sein. Dadurch kann sichergestellt werden, dass der Arbeitsbereich der Verstärker bzw. der Differenzverstärker nahe an dem Referenzpotenzial liegt. Alternativ kann der Knotenpunkt über den zweiten elektrischen Widerstand auch mit einer anderen Masse verbunden sein, etwa mit Gehäusemasse oder mit der Masse der Platine.

Die ersten elektrischen Widerstände, die mit der ersten Anzahl von Elektroden verbunden sind, können hochohmig ausgestaltet sein.

In einer Ausgestaltung der Erfindung können die Mittel zum Bereitstellen eines gemittelten elektrischen Referenzpotenzials eine Elektrode umfassen, wobei das gemittelte elektrische Referenzpotenzial durch eine Mittelung über die Zeit des an dieser Elektrode detektierten elektrischen Potenzials gebildet wird.

### Kurzbeschreibung der Figuren

Weitere Einzelheiten und Merkmale der Erfindung sowie konkrete Ausführungsbeispiele der Erfindung ergeben sich aus der nachfolgenden Beschreibung in Verbindung mit der Zeichnung. Es zeigt:
- Fig. 1: ein Ausführungsbeispiel der Erfindung in Form einer Elektrodenmatte, die in oder auf ein Gehirn implantiert werden kann und auf der eine Anzahl von Ableitelektroden und eine Anzahl von Referenzelektroden aufgebracht sind; und
- Fig. 2: eine alternative Ausgestaltung einer erfindungsgemäßen Sensoreinrichtung, bei der keine eigenen Referenzelektroden notwendig sind.

### Detaillierte Beschreibung der Erfindung

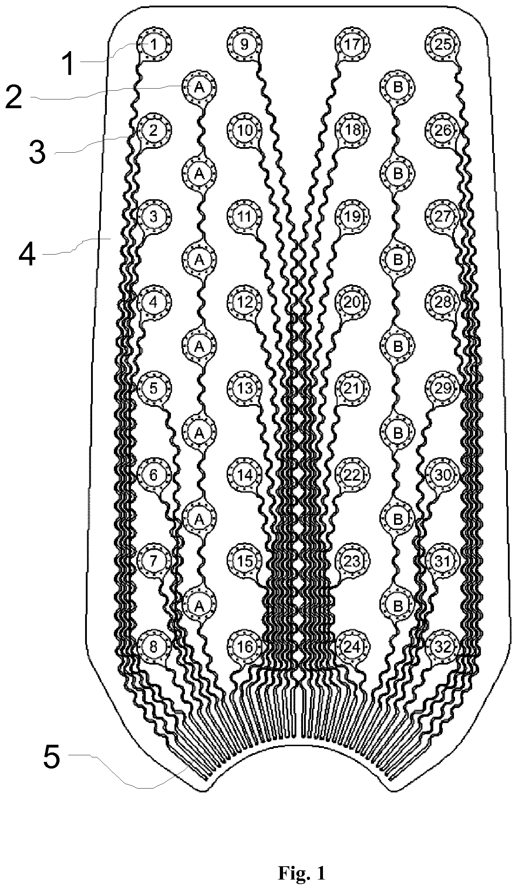

**Fig. 1** zeigt ein erstes Ausführungsbeispiel der Erfindung in Form einer Elektrodenmatte, auf der eine Anzahl von Elektroden bzw. Ableitelektroden 1 (die Ableitelektroden sind hier von 1 bis 32 durchnummeriert) und zwei Referenzelektroden 2 (die Referenzelektroden sind mit den Buchstaben A und B gekennzeichnet) angeordnet sind.

Die Ableitelektroden 1 sind jeweils über Leiterbahnen 3 mit Anschlüssen verbunden, die sich im Anschlussbereich 5 der Elektrodenmatte befinden. Gemäß Fig. 1 sind auf der Elektrodenmatte zwei Referenzelektroden 2 (A und B) angeordnet, wobei jede Referenzelektrode aus sieben Einzelkontakten gebildet wird, die über Leiterbahnen miteinander verbunden sind.

Die Ableitelektroden 1, die Referenzelektroden 2, die Leiterbahnen der Ableitelektroden 1 und die die Einzelkontakte der Referenzelektroden verbindenden Leiterbahnen sind in ein elektrisch isolierendes Substrat der Elektrodenmatte eingebettet. Das elektrisch isolierende Substrat weist in den Bereichen der Ableitelektroden bzw. der Einzelkontakte der Referenzelektroden Öffnungen auf, sodass die Ableitelektroden und die Einzelkontakte der Referenzelektroden mit einem biologischen Organismus, etwa mit den Nervenzellen eines Nervensystems gekoppelt werden können.

In dem hier gezeigten Ausführungsbeispiel sind die Leiterbahnen mäanderförmig ausgestaltet, sodass eine mechanische Dehnung der Elektrodenmatte möglich ist, ohne dass die Gefahr eines Leiterbahnbruches besteht.

Die Einzelkontakte der Referenzelektrode 2 sind so zwischen den Ableitelektroden 1 angeordnet, dass sie jeweils immer einen im Wesentlichen gleich großen Abstand zu den Ableitelektroden 1 aufweisen. Durch die galvanische Verbindung der Einzelkontakte einer Referenzelektrode 2 werden die an den einzelnen Einzelkontakten detektierten Potenziale gemittelt, sodass an dem Anschluss einer Referenzelektrode ein gemitteltes Referenzpotenzial bereitgestellt wird. Vorteilhaft ist hierbei, dass nur mehr ein Anschlusskontakt bereitgestellt werden muss, um etwa das gemittelte Referenzpotenzial an eine körperexterne Verarbeitungseinheit zu übertragen. Die Anzahl der an eine körperexterne Verarbeitungseinheit zu übertragenden Signale kann so erheblich reduziert werden. Das bereitgestellte gemittelte Referenzpotenzial kann verwendet werden, um zwischen dem gemittelten Referenzpotenzial und einem an einer Ableitelektrode 1 detektierten elektrischen Potenzial eine elektrische Potenzialreferenz zu detektieren und diese gegebenenfalls mit Hilfe eines Verstärkers, etwa Differenzialverstärker, zu verstärken.

Weil die Einzelkontakte der Referenzelektroden 2 zu jeder Ableitelektrode 1 jeweils einen unterschiedlichen Abstand aufweisen, ist gewährleistet, dass die aus den Einzelkontakten gebildete Referenzelektrode unempfindlich gegen lokale bioelektrische Signale im Bereich einer einzelnen Ableitelektrode ist. Wie aus Fig. 1 ersichtlich, ist dennoch gewährleistet, dass an jeder Ableitelektrode 1 in unmittelbarer Nachbarschaft ein Einzelkontakt einer Referenzelektrode 2 angeordnet ist, sodass die Eingangs genannte Anforderung, nach der eine Ableitelektrode und eine Referenzelektrode möglichst dicht beieinander liegen sollen, erfüllt ist. Weil jeder Einzelkontakt einer Referenzelektrode 2 einen verschiedenen Abstand zu einer bestimmten Ableitelektrode aufweist, ist jedenfalls für den am weitesten zur Ableitelektrode beabstandeten Einzelkontakt der Referenzelektrode 2 die Eingangs genannte zweite Anforderung erfüllt, nach der eine Referenzelektrode und eine Ableitelektrode möglichst weit voneinander entfernt liegen sollen.

Selbstverständlich sind auch andere Varianten einer Anordnung von Ableitelektroden und Referenzelektroden auf einer Elektrodenmatte möglich, als die in Fig. 1 gezeigte spaltenförmige Anordnung.

Ebenso können auch mehr oder weniger als die in Fig. 1 gezeigten 32 Ableitelektroden und mehr oder weniger als die in Fig. 1 gezeigten sieben Einzelkontakte der Referenzelektroden oder auch mehr als zwei Referenzelektroden vorgesehen sein.

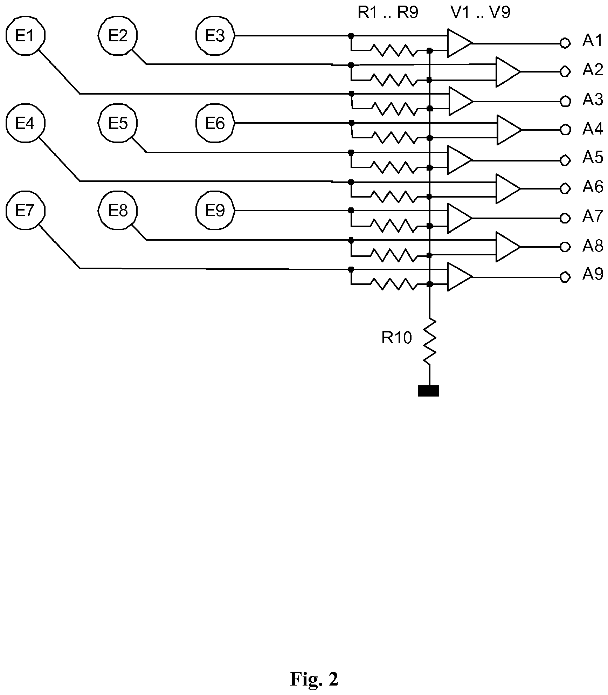

Fig. 2 zeigt ein weiteres Ausführungsbeispiel einer erfindungsgemäßen Sensoreinrichtung zur Detektion bioelektrischer Signale, insbesondere zur Implantation in oder auf ein Gehirn.

Gezeigt ist in Fig. 2 ein Neun-Kanal-Elektrodenarray, welches neun Ableitelektroden E1 bis E9 aufweist. Ferner weist die in Fig. 2 gezeigte Sensoreinrichtung für jeden Kanal einen Verstärker bzw. Differenzverstärker V1 bis V9 auf. Die Ableitelektroden E1 bis E9 sind jeweils über einen Kontakt direkt mit einem ersten Eingang jeweils eines Differenzverstärkers V1 bis V9 gekoppelt. Ferner sind alle Ableitelektroden E1 bis E9 über jeweils einen hochohmigen Widerstand R1 bis R9 miteinander verbunden. Beispielsweise können Widerstände von etwa 10 MΩ vorgesehen sein.

Der Verbindungspunkt bzw. Knotenpunkt der elektrischen Widerstände R1 bis R9 bildet die Referenz für sämtliche Differenzverstärker V1 bis V9, d.h., an dem Knotenpunkt der elektrischen Widerstände R1 bis R9 wird ein Referenzpotenzial für die Differenzverstärker abgegriffen. Aus elektrischer Sicht entspricht das an dem Knotenpunkt der elektrischen Widerstände R1 bis R9 abgegriffene elektrische Potenzial dem Durchschnittspotenzial aller Ableitelektroden E1 bis E9.

Um sicherzustellen, dass der Arbeitsbereich der Verstärker V1 bis V9 nahe an dem Referenzpotenzial liegt, wird der Knotenpunkt bzw. Verbindungspunkt über einen zweiten elektrischen Widerstand, der beispielsweise im Bereich einiger kΩ liegen kann, mit dem Massepotenzial der Versorgungsspannung der Differenzverstärker V1 bis V9 verbunden. Alternativ kann der zweite elektrische Widerstand auch mit einer anderen Masse verbunden sein.

Nach Verstärkung der jeweiligen Potenzialdifferenzen liegen an den Ausgängen A1 bis A9 der Differenzverstärker die von den Ableitelektroden E1 bis E9 detektierten Signale in verstärkter Form vor.

Die Ausführungsform gemäß Fig. 2 hat den Vorteil, dass sämtliche Elektroden der Sensoreinrichtung zum elektrischen Ableiten bzw. zur Detektion von bioelektrischen Signalen verwendet werden können. Es müssen keine eigenen bzw. zusätzlichen Referenzelektroden vorgesehen werden, denn die Ableitelektroden E1 bis E9 werden in Kombination mit den elektrischen Widerständen R1 bis R9 auch als Referenzelektroden verwendet, wobei die Ableitelektroden E1 bis E9 über die Widerstände R1 bis R9 zu einer einzelnen Referenzelektrode zusammengeschaltet sind. Dadurch kann die auf einer Elektrodenmatte zur Verfügung stehende Fläche optimal ausgenutzt werden, da anstelle von separaten Referenzelektroden bzw. Elektrodenkontakten einer Referenzelektrode zusätzliche Ableitelektroden angeordnet werden können.

Die hier gezeigte Sensoreinrichtung ist insbesondere vorgesehen, um in oder auf ein Gehirn implantiert zu werden und dort insbesondere mit Nervenzellen eines Nervensystems gekoppelt zu werden.

### Bezugszeichen:

- 1: Elektroden bzw. Ableitelektroden 1 bis 32
- 2: Referenzelektroden A und B
- 3: Leiterbahnen
- 4: elektrisch isolierendes Substrat
- 5: Anschlussbereich
- E1 bis E9: Elektroden bzw. Ableitelektroden
- R1 bis R9: elektrische Widerstände
- R10: elektrischer Widerstand zum Massepotenzial der Verstärker
- V1 bis V9: Verstärker (Differenzverstärker)
- A1 bis A9: Anschlüsse (Elektrodensignale nach Verstärkung)

## Patentansprüche

1. Sensoreinrichtung zur Detektion bioelektrischer Signale, zur Implantation in oder auf ein Gehirn, wobei die Sensoreinrichtung eine erste Anzahl von Elektroden (1), die mit einem biologischen Organismus, insbesondere Nervenzellen eines Nervensystems koppelbar sind, zum Abgreifen einer ersten Anzahl elektrischer Potenziale an dem biologischen Organismus umfasst, wobei
die Sensoreinrichtung Mittel zum Bereitstellen eines gemittelten elektrischen Referenzpotenzials umfasst,
die Mittel zum Bereitstellen eines gemittelten elektrischen Referenzpotenzials in die Sensoreinrichtung integriert sind und mit der Sensoreinrichtung implantierbar sind,
**dadurch gekennzeichnet, dass**
die Sensoreinrichtung eine erste Anzahl von Differenzverstärkern umfasst, wobei ein erster Eingang eines jeden Differenzverstärkers jeweils mit einer Elektrode (1) ist und ein zweiter Eingang mit dem Mittel zum Bereitstellen eines gemittelten elektrischen Referenzpotenzials verbunden ist, und
die Mittel zum Bereitstellen eines gemittelten elektrischen Referenzpotenzials eine erste Anzahl von ersten elektrischen Widerständen umfassen, wobei jede Elektrode (1) mit jeweils einen der ersten elektrischen Widerstände verbunden ist und die ersten elektrischen Widerstände ihrerseits in einem Kontenpunkt miteinander verbunden sind, der das gemittelte elektrische Referenzpotenzial bereitstellt.

2. Sensoreinrichtung nach Anspruch 1, **dadurch gekennzeichnet, dass** die Mittel zum Bereitstellen eines gemittelten elektrischen Referenzpotenzials eine zweite Anzahl von Referenzelektroden (2) umfassen, die galvanischen Verbindungen mechanisch dehnbare Verbindungen umfassen.

3. Sensoreinrichtung nach Anspruch 1 oder 2, **dadurch gekennzeichnet, dass** die Mittel zum Bereitstellen eines gemittelten elektrischen Referenzpotenzials eine zweite Anzahl von Referenzelektroden (2) umfassen, die galvanischen Verbindungen mäanderförmige Leiterbahnen umfassen.

4. Sensoreinrichtung nach einem der Ansprüche 1 bis 3, **dadurch gekennzeichnet, dass** die Referenzelektroden (2) so relativ zu den Elektroden (1) angeordnet sind, dass die Abstände zwischen einer Elektroden (1) und den Referenzelektroden im Wesentlichen verschieden sind.

5. Sensoreinrichtung nach einem der vorherigen Ansprüche, **dadurch gekennzeichnet, dass** die ersten elektrischen Widerstände hochohmig sind.

## Claims

1. Sensor device for detecting bioelectric signals and for implanting in or on a brain, the sensor device comprising a first number of electrodes (1) which can be coupled to a biological organism, in particular nerve cells of a nervous system, for tapping a first number of electrical potentials on the biological organism, the sensor device comprising means for providing an averaged electrical reference potential, the means for providing an averaged electrical reference potential being integrated into the sensor device and being implantable together with the sensor device, **characterized in that** the sensor device comprises a first number of differential amplifiers, a first input of each differential amplifier being connected to an electrode (1) in each case and a second input being connected to the means for providing an averaged electrical reference potential, and the means for providing an averaged electrical reference potential comprise a first number of first electrical resistors, each electrode (1) being connected to one of the first electrical resistors in each case and the first electrical resistors in turn being interconnected at an node which provides the averaged electrical reference potential.

2. Sensor device according to claim 1, **characterized in that** the means for providing an averaged electrical reference potential comprise a second number of reference electrodes (2); the galvanic connections comprise mechanically stretchable connections.

3. Sensor device according to either claim 1 or claim 2, **characterized in that** the means for providing an averaged electrical reference potential comprise a second number of reference electrodes (2); the galvanic connections comprise meandering conductor tracks.

4. Sensor device according to any of claims 1 to 3, **characterized in that** the reference electrodes (2) are arranged relative to the electrodes (1) such that the distances between an electrode (1) and the reference electrodes are substantially different.

5. Sensor device according to any of the preceding claims, **characterized in that** the first electrical resistors have high impedance.

## Revendications

1. Dispositif capteur pour la détection de signaux bioélectriques, destiné à être implanté dans ou sur un cerveau, le dispositif capteur comprenant un premier nombre d'électrodes (1) pouvant être couplées à un organisme biologique, en particulier aux neurones d'un système nerveux, pour détecter un premier nombre de potentiels électriques à partir de l'organisme biologique,
le dispositif capteur comprenant des moyens pour fournir un potentiel électrique de référence moyenné,
les moyens pour fournir un potentiel électrique de référence moyenné étant intégrés dans le dispositif capteur et pouvant être implantés à l'aide du dispositif capteur,
**caractérisé en ce que**
le dispositif capteur comprend un premier nombre d'amplificateurs différentiels, une première entrée de chaque amplificateur différentiel étant reliée à une électrode (1) et une seconde entrée étant reliée au moyen pour fournir un potentiel électrique de référence moyenné, et
les moyens pour fournir un potentiel électrique de référence moyenné comprennent un premier nombre de premières résistances électriques, chaque électrode (1) étant reliée à l'une des premières résistances électriques, et les premières résistances électriques étant à leur tour reliées les unes aux autres en un nœud qui fournit le potentiel électrique de référence moyenné.

2. Dispositif capteur selon la revendication 1, **caractérisé en ce que** les moyens pour fournir un potentiel électrique de référence moyenné comprennent un second nombre d'électrodes de référence (2), les liaisons galvaniques comprenant des liaisons extensibles mécaniquement.

3. Dispositif capteur selon la revendication 1 ou 2, **caractérisé en ce que** les moyens pour fournir un potentiel électrique de référence moyenné comprennent un second nombre d'électrodes de référence (2), les liaisons galvaniques comprenant des lignes conductrices en forme de méandres.

4. Dispositif capteur selon l'une des revendications 1 à 3, **caractérisé en ce que** les électrodes de référence (2) sont disposées par rapport aux électrodes (1) de telle sorte que les distances entre une électrode (1) et les électrodes de référence sont sensiblement différentes.

5. Dispositif capteur selon l'une des revendications précédentes, **caractérisé en ce que** les premières résistances électriques sont à haute résistance ohmique.
